(19) Europäisches Patentamt
European Patent Office
Office européen des brevets

(11) EP 0 579 797 B1

(12) EUROPÄISCHE PATENTSCHRIFT

(45) Veröffentlichungstag und Bekanntmachung des Hinweises auf die Patenterteilung:
**21.04.1999 Patentblatt 1999/16**

(21) Anmeldenummer: **93902021.0**

(22) Anmeldetag: **01.02.1993**

(51) Int Cl.$^6$: **C07F 9/50**
// C07F9/53

(86) Internationale Anmeldenummer:
**PCT/CH93/00026**

(87) Internationale Veröffentlichungsnummer:
**WO 93/15091 (05.08.1993 Gazette 1993/19)**

# (54) DIPHOSPHINLIGANDEN

DIPHOSPHINE LIGANDS

LIGANDS DIPHOSPHINIQUES

(84) Benannte Vertragsstaaten:
**AT BE CH DE DK ES FR GB IT LI NL**

(30) Priorität: **31.01.1992 CH 289/92**
**16.04.1992 CH 1270/92**
**18.05.1992 CH 1582/92**
**19.06.1992 CH 1944/92**

(43) Veröffentlichungstag der Anmeldung:
**26.01.1994 Patentblatt 1994/04**

(73) Patentinhaber: **F. HOFFMANN-LA ROCHE AG**
**4002 Basel (CH)**

(72) Erfinder:
- **FORICHER, Joseph**
  **F-68200 Mulhouse (FR)**
- **SCHMID, Rudolf**
  **CH-4144 Arlesheim (CH)**

(74) Vertreter: **Cottong, Norbert A.**
**F.Hoffmann-La Roche AG**
**Patent Department (PLP),**
**124 Grenzacherstrasse**
**4070 Basel (CH)**

(56) Entgegenhaltungen:
**EP-A- 0 104 375**
**EP-A- 0 398 132**
**EP-A- 0 408 338**
**WO-A-92/16535**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 579 797 B1

## Beschreibung

Die vorliegende Erfindung betrifft neue, racemische und optisch aktive Phosphorverbindungen der allgemeinen Formel

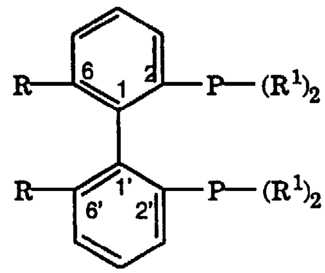

I

worin R $C_1$-$C_5$ Alkyl oder $C_1$-$C_5$ Alkoxy bedeutet und $R^1$ $C_1$-$C_5$ Alkyl oder mit Phenyl oder Trialkylsilyl substituiertes Phenyl darstellt.

Die Erfindung betrifft ferner die Herstellung der Phosphorverbindungen der Formel I, sowie deren Verwendung für enantioselektive Reaktionen, wie z.B. asymmetrische Hydrierungen, enantioselektive Wasserstoffverschiebungen in prochiralen, allylischen Systemen, und dergleichen.

In der europäischen Patentanmeldung EP-A 0 398 132 werden Di-phenylphosphin- oder Di-p-tolylphosphin-Verbindungen beschrieben wie z.B. (6,6-Dimethoxybiphenyl-2,2'-diyl)bis(diphenylphosphin) oder (6,6-Dimethoxybiphenyl-2,2'-diyl)bis(di-p-tolylphosphin).

In der europäischen Patentanmeldung EP-A 0 104 375 werden Di-phenylphosphin-Verbindungen beschrieben wie z.B. 6,6-Dimethylbiphenyl-2,2'-diyl)bis(diphenylphosphin)

In der europäischen Patentanmeldung EP-A 0 408 338 werden asymmetrische Synthesen von Carbonsäuren und Estern aus den jeweiligen ungesättigten Vorstufen mittels einer Diphosphinverbindung des Typs 2,2'-bis (Dicyclohexylphosphinyl)-6,6'-dimethyl-1,1'-biphenyl beschrieben.

In der internationalen Patentanmeldung WO-A-92 16535 werden Diphosphin-Verbindungen als Zwischenprodukte zur Herstellung von Phosphonsäurediphenylestern beschrieben.

Keine der oben genannten Patentanmeldungen beschreibt Phosphorverbindungen der Formel I, worin $R^1$ niederes Alkyl oder durch Phenyl oder durch Trialkylsilyl substituiertes Phenyl bedeutet.

Der Ausdruck "$C_1$-$C_5$ Alkyl" bedeutet, im Rahmen der vorliegenden Erfindung, geradkettige oder verzweigte Alkylgruppen mit 1 bis 5 Kohlenstoffatomen wie Methyl, Aethyl, Propyl, Isopropyl, Butyl, Isobutyl, tert. Butyl, Pentyl, Isopentyl und dergleichen. Der Ausdruck "$C_1$-$C_5$ Alkoxy" bedeutet Gruppen, in denen der Alkylrest die vorhergehende Bedeutung hat., Der Ausdruck " mit Phenyl oder Trialkylsilyl substituiertes Phenyl" bedeutet, im Rahmen der vorliegenden Erfindung, den Phenylrest, welcher in meta- oder para-Stellung oder auch meta,meta-substituiert sein kann. Als Substituenten kommen hier in Frage Phenyl, Trialkylsilyl wie Trimethylsilyl, Triäthylsilyl, und dergleichen.

Die Phosphorverbindungen der Formel I können sowohl in racemischer als auch in optisch aktiver Form vorliegen. Bevorzugte Verbindungen der Formel I sind solche, worin R Methoxy oder Methyl, insbesondere Methoxy bedeutet. Ferner sind auch noch diejenigen bevorzugt, worin $R^1$ Isopropyl, Isopentyl, oder substituiertes Phenyl bedeutet. Hierbei ist der Phenylring bevorzugt in para- oder meta,meta-Stellung substituiert und zwar mit Phenyl, Trimethylsilyl oder auch Triäthylsilyl. Besonders bevorzugte Verbindungen der Formel I sind:

(R)- oder (S)-(6,6'-Dimethoxybiphenyl-2,2'-diyl)bis(di-4-biphenylylphosphin).
(R)- oder (S)-(6,6'-Dimethoxybiphenyl-2,2'-diyl)bis[bis-[4-(trimethylsilyl)phenyl]phosphin].
(R)- oder (S)-(6,6'-Dimethoxybiphenyl-2,2'-diyl)bis[di-(metaterphenyl-5'-yl)phosphin.
(R)- oder (S)-(6,6'-Dimethoxybiphenyl-2,2'-diyl)bis[bis(3,5-bis- (trimethylsilyl)phenyl)phosphin].
(R)- oder (S)-(6,6'-Dimethoxybiphenyl-2,2'-diyl)bis[bis(3,5-bis-(triäthylsilyl)phenyl)phosphin].
(R)- oder (S)-(6,6'-Dimethoxybiphenyl-2,2'-diyl)bis(diisopropylphosphin).
(R)- oder (S)-(6,6'-Dimethoxybiphenyl-2,2'-diyl)bis(diäthylphosphin).

Die erfindungsgemässen Verbindungen der Formel I können in an sich bekannter Weise hergestellt werden. Dies kann z.B. ausgehend von Verbindungen der allgemeinen Formel II erfolgen

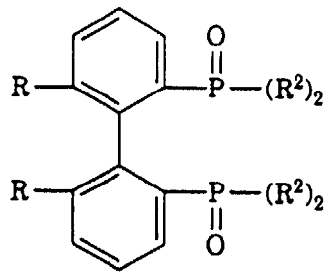

II

worin R die obige Bedeutung hat und $R^2$ Phenoxy, Chlor oder Brom darstellt.

Die Herstellung erfolgt hierbei z.B. durch Umsetzung einer Verbindung der Formel II mit einer Grignard- oder Lithium-Verbindung der Formel

$$R^1MgX \quad \text{bzw.} \quad R^1Li$$

worin X Chlor, Brom oder Jod darstellt,
wobei man eine Verbindung der Formel III erhält,

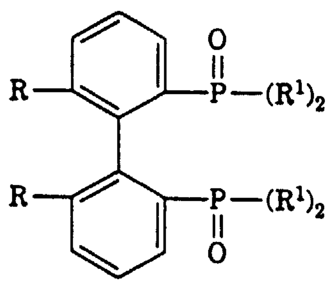

III

worin R und $R^1$ die obige Bedeutung haben,
welche anschliessend reduziert wird zu einer Verbindung der Formel I.

Die Umsetzung einer Verbindung der Formel II mit $R^1MgX$ oder $R^1Li$ kann in an sich bekannter Weise erfolgen. Vorzugsweise erfolgt dies z.B. unter den üblichen Bedingungen einer Grignard-Reaktion. Hierbei werden vorzugsweise Verbindungen der Formel II, worin $R^2$ Phenoxy darstellt, mit einer Verbindung der Formel $R^1MgX$ umgesetzt, und solche, worin $R^2$ Chlor darstellt, mit einer Verbindung der Formel $R^1$-Li oder $R^1MgX$.

Die Reduktion einer racemischen oder in (R)- oder (S)-Form vorliegenden Verbindung der Formel III kann in an sich bekannter Weise durchgeführt werden. Dies kann beispielsweise mit Silanen, wie z.B. Trichlorsilan, in einem aromatischen Kohlenwasserstoff, wie etwa in siedendem Xylol, oder auch in Acetonitril usw., zweckmässig in Gegenwart einer Hilfsbase, wie etwa Triäthylamin, oder vorzugsweise Tributylamin, erfolgen. Gewünschtenfalls kann diese Reduktion auch in einem Autoklaven unter Druck durchgeführt werden.

Die als Ausgangsmaterial verwendeten Verbindungen der Formel II sind bekannte Verbindung und können z.B. gemäss WO 92/16535 hergestellt werden.

Die erfindungsgemässen Phosphorverbindungen der Formel I bilden Komplexe mit Uebergangsmetallen wie etwa Metallen der Gruppe VIII, insbesondere mit Ruthenium, Rhodium und Iridium, welche als Katalysatoren bei asymmetrischen Hydrierungen und auch für enantioselektive Wasserstoffverschiebungen in prochiralen, allylischen Systemen verwendbar sind. Für die erwähnten Hydrierungen sind Ruthenium- und Rhodium-Komplexe bevorzugt, während für Isomerisierungen Rhodium-Komplexe bevorzugt sind. Diese Katalysatoren, d.h. die Komplexe aus einem Metall der Gruppe VIII und den Phosphorverbindungen der Formel I sind neu und ebenfalls Gegenstand der vorliegenden Erfindung.

Die in Frage stehenden Komplexe können in an sich bekannter Weise hergestellt werden, z.B. indem man eine Verbindung der Formel I mit einer Verbindung, welche ein Metall der Gruppe VIII abgeben kann, in einem geeigneten, inerten organischen oder wässrigen Lösungsmittel umsetzt. Als geeignete, z.B. Rhodium abgebende Verbindungen können beispielsweise genannt werden, organische Rhodium-Komplexe mit Aethylen, Propylen und dergleichen, sowie mit bis-Olefinen, z.B. (Z,Z)-1,5-Cyclooctadien, 1,5-Hexadien, Bicydo[2.2.1]-hepta-2,5-dien oder mit weiteren Dienen, welche mit Rhodium leicht lösliche Komplexe bilden. Bevorzugte, Rhodium abgebende Verbindungen

sind z.B. Di-μ-chloro-bis[$\eta^4$-(Z,Z)-1,5-cyclooctadien]dirhodium(I), Di-μ-chloro-bis[$\eta^4$-norbornadien]dirhodium(I), Di-μ-Trifluoracetato-bis[$\eta^4$-(Z,Z)-1,5-cyclooctadien]dirhodium(I), Bis[$\eta^4$,(Z,Z)-1,5-cyclooctadien]rhodium-tetrafluorborat oder Bis[$\eta^4$-(Z,Z)-cyclooctadien]rhodium-perchlorat. Als Iridium abgebende Verbindung kann beispielsweise Di-μ-chloro-bis[$\eta^4$-(Z,Z)-1,5-cyclooctadien]diiridium(I) genannt werden.

Die in Frage stehenden Ruthenium-Komplexe können z.B. durch folgende Formel dargestellt werden

$$Ru(Z)_2L \qquad IV$$

worin Z Halogen oder die Gruppe A-COO, A $C_1$-$C_5$ Alkyl, Aryl, halogeniertes $C_1$-$C_5$ Alkyl oder halogeniertes Aryl und L einen chiralen Diphosphinliganden der Formel I darstellen.

Diese Komplexe können im Prinzip in an sich bekannter Weise hergestellt werden. Zweckmässig und bevorzugt werden Ruthenium-Komplexe beispielsweise dadurch hergestellt, dass man einen Komplex der Formel

$$[Ru(Z^1)_2L^1{}_m]_p \cdot (H_2O)_q \qquad V$$

worin $Z^1$ Halogen oder eine Gruppe $A^1$-COO, $A^1$ C1-C5 Alkyl oder halogeniertes C1-C5 Alkyl, $L^1$ einen neutralen Liganden, m die Zahl 1, 2 oder 3, p die Zahl 1 oder 2 und q die Zahl 0 oder 1 darstellen, mit einem chiralen Diphosphinliganden der Formel I umsetzt, oder, dass man einen Ruthenium-Komplex der Formel

$$Ru(CF_3COO)_2L \qquad VI$$

worin L einen chiralen Diphosphinliganden der Formel I darstellt, mit einem das Anion Z abgebenden Salz, worin Z obige Bedeutung hat, umsetzt.

Der Ausdruck "neutraler Ligand" bedeutet, im Rahmen der vorliegenden Erfindung, einen leicht austauschbaren Liganden wie etwa ein Diolefin, z.B. Norbornadien, (Z,Z)-1,5-Cyclooctadien usw., oder auch ein Nitril wie Acetonitril, Benzonitril und dergleichen. Falls m die Zahl 2 oder 3 darstellt, können die Liganden gleich oder auch verschieden sein.

Die Rutheniumkomplexe der Formel V sind bekannte Substanzen oder Analoge bekannter Substanzen, welche leicht in zur Herstellung der der bekannten analoger Weise erhalten werden können, beispielsweise gemäss Albers, M.O. et al., J. Organomet. Chem. 272, C62-C66 (1984).

Die Umsetzung eines Rutheniumkomplexes der Formel V mit einem chiralen Diphosphinliganden der Formel I kann in an sich bekannter Weise durchgeführt werden. Diese Reaktion kann zweckmässig in einem inerten organischen Lösungsmittel erfolgen. Als Beispiele derartiger Lösungsmittel können genannt werden, z.B. Aether wie Tetrahydrofuran oder Dioxan, Ketone wie etwa Aceton, niedere Alkohole wie etwa Methanol, Aethanol usw., halogenierte Kohlenwasserstoffe wie Methylenchlorid, Chloroform und dergleichen, oder auch Gemische derartiger Lösungsmittel. Die Reaktion kann zudem bei einer Temperatur zwischen etwa 0°C und etwa 100°C, und vorzugsweise zwischen etwa 15°C und etwa 60°C erfolgen, jedoch unter striktem Ausschluss von Sauerstoff.

Die Umsetzung eines Rutheniumkomplexes der Formel VI (erhältlich aus einem Komplex der Formel V) mit einem das Anion Z enthaltenden Salz kann in an sich bekannter Weise erfolgen. Der Ausdruck "ein das Anion Z abgebendes Salz" bedeutet im Rahmen der vorliegenden Erfindung beispielsweise Ammoniumsalze, Alkalimetallsalze oder andere geeignete Metallsalze. Um die Löslichkeit derartiger Salze zu verbessern, können in gewissen Fällen auch Kronenäther oder dergleichen zugesetzt werden.

Wie bereits eingangs erwähnt, sind die erfindungsgemässen Phosphorverbindungen, in Form von Komplexen mit Metallen der Gruppe VIII, und insbesondere Rhodium und Ruthenium, u.a. verwendbar für asymmetrische Hydrierungen. Als besonders geeignete Substrate können in diesem Zusammenhang insbesondere genannt werden, Allylalkohole wie z.B. Geraniol, 6,7-Dihydrogeraniol, 6,7-Dihydrofarnesol, 6,7,10,11-Tetrahydrofarnesol und dergleichen, Enamide wie z.B. (Z)-2-Acetyl-1-(p-methoxybenzyliden-1,2,3,4,5,6,7,8-octahydroisochinolin, α,β-ungesättigte Säuren wie z.B. 3,4,6,11-Tetra-hydro-6,11-dioxo-pyridazo[1,2-a]phthalazin-1-carbonsäure sowie auch funktionalisierte Ketone wie β-Ketoester, z.B. Acetessigsäuremethyl-oder äthylester usw. oder auch 2-Pyridylketone wie z.B. 2-Acetylpyridin, 2-Pyridyl-2,8-bis(trifluormethyl)-4-chinolylketon und dergleichen.

Bei der Durchführung derartiger Hydrierungen, können diese Komplexe zuerst hergestellt und dann zu einer Lösung der zu hydrierenden Substanz gegeben werden. Alternativ können sie jedoch auch in situ hergestellt werden, z.B. auch in Gegenwart einer zu hydrierenden Substanz.

Die asymmetrische Hydrierung kann in einem geeigneten, unter den Reaktionsbedingungen inerten Lösungsmittel erfolgen. Als derartige Lösungsmittel können insbesondere genannt werden, aromatische Kohlenwasserstoffe wie Benzol, Toluol usw., niedere Alkohole wie z.B. Methanol oder Aethanol, Wasser, Ester wie Aethylacetat, halogenierte Kohlenwasserstoffe wie Methylenchlorid, Chloroform und dergleichen, cyclische Aether wie Tetrahydrofuran oder Dioxan, und dergleichen, oder Gemische derartiger Lösungsmittel.

Das Verhältnis von Metall zu Ligand L liegt zweckmässig zwischen etwa 0,05 und etwa 5 Mol, bzw. zwischen etwa 0,5 und etwa 2 Mol, vorzugsweise bei etwa 1 Mol Metall pro Mol Ligand. Das Verhältnis von Metall, in den Komplexen wie z.B. der Formel IV, zu den zu hydrierenden Substanzen liegt zweckmässig zwischen etwa 0,0005 und etwa 1 Mol %, vorzugsweise zwischen etwa 0,002 und etwa 0,1 Mol %.

Die asymmetrische Hydrierung mit Komplexen wie z.B. der Formel IV erfolgt zweckmässig bei einer Temperatur von etwa 0°C bis etwa 150°C, in Abhängigkeit des verwendeten Substrates. Diese Hydrierung erfolgt zweckmässig auch unter Druck, vorzugsweise bei einem Druck von etwa 2 bis etwa 200 bar, besonders bevorzugt von etwa 10 bis etwa 100 bar.

Sämtliche vorhergehend erwähnten Reaktionen werden zweckmässig unter Inertgas, wie z.B. Argon oder Stickstoff, durchgeführt.

Die folgenden Beispiele dienen zur Illustrierung der Erfindung und stellen in keiner Weise irgendeine Beschränkung dar. In diesen Beispielen haben die gewählten Abkürzungen folgende Bedeutung:

DC Dünnschichtchromatographie
GC Kapillar-Gaschromatographie
e.e. Enantiomeric Excess.
THF Tetrahydrofuran
Ar Argon
RT Raumtemperatur

Alle Temperaturen sind in °Celsius angegeben.

Beispeil 1

Synthese von (R)- und (S)-(6,6"Dimethoxybiphenyl-2,2'-diyl)bis(di-4-biphenylylphosphin) [(R)- und (S)-p-Biphenyl-MeOBIPHEP]

Zu 6.78 g (10 mMol) (6,6'-Dimethoxybiphenyl-2,2'-diyl)bis(phosphonsäure-diphenylester) wurde eine aus 3.0 g (123 mMol) Magnesiumspänen und 23.3 g (100 mMol) 4-Brombiphenyl in 250 ml trockenem THF zubereitete Lösung von 4-Biphenylylmagnesiumbromid bei RT getropft. Die erhaltene Lösung wurde noch 30 Min. bei RT und 1 Stunde bei 40° gerührt und 2 Stunden bei 65° gekocht. Nach Abkühlung auf 0° wurde die Reaktionslösung langsam mit einer $NH_4Cl$-Lösung versetzt. Die organische Phase wurde abgetrennt, mit ges. NaCl-Lösung gewaschen, über $MgSO_4$ getrocknet, filtriert und eingedampft. Der Rückstand wurde an 500g Kieselgel chromatographiert (Elution mit Essigsäureäthylester, dann $CH_2Cl_2$/Aethanol 9:1, dann Aethanol). Das erhaltene Produkt (8.9g) wurde aus Essigsäureäthylester/Hexan umkristallisiert. Das Kristallisat wurde abgenutscht, mit Hexan gewaschen und im HV (~10 Pa) bei 100°C getrocknet. Es wurden 7.0 g (R)-(6,6'-Dimethoxybiphenyl-2,2'-diyl)bis(di-4-biphenylylphosphinoxid) als weisses Pulver erhalten; Smp. 190-210°C; $[\alpha]_D^{20}$ = +67.5 (c=1.0, $CHCl_3$).

Zu einer Lösung von 6.87 g (7.47 mMol) (R)-(6,6'-Dimethoxybiphenyl-2,2'-diyl)bis(di-4-biphenylphosphinoxid) in 80 ml trockenem Xylol und 13 ml trockenem Tributylamin wurden unter Ar und unter Kühlung 5 ml Trichlorsilan getropft. Die Reaktionslösung wurde 3 Stunden unter Ar am Rückfluss gekocht. Nach Abkühlung wurde das Reaktionsgemisch unter Ar via eine Stahlkanüle in 100 ml deoxygenierte 30% NaOH-Lösung eingetragen, wobei die Temperatur bis auf 60-70° anstieg. Das Zweiphasengemisch wurde noch 1 Stunde bei 60-70° gerührt, und nach Abkühlung wurden die Phasen getrennt. Die organische Phase wurde mit Wasser (2 x 100 ml) und mit ges. NaCl-Lösung (2 x 100 ml) gewaschen, über $MgSO_4$ getrocknet, filtriert und am Rotationsverdampfer eingeengt. Der Rückstand wurde am HV (~10 Pa) bei 80° getrocknet und aus Aethanol/$CH_2Cl_2$ umkristallisiert. Nach Trocknen am HV (~10 Pa) bei 100° wurden 6.5 g (R)-(6,6'-Dimethoxybiphenyl-2,2'-diyl)bis(di-4-biphenylylphosphin) als weisses Pulver vom Smp. 217-218° isoliert; $[\alpha]_D^{20}$ = +39 (c=1.0, $CHCl_3$).

In zum Vorhergehenden analoger Weise wurden folgende Verbindungen hergestellt:

(S)-(6,6'-Dimethoxybiphenyl-2,2'-diyl)bis(di-4-biphenylylphosphinoxid); Smp. 230-245°; $[\alpha]_D^{20}$ = -55.8 (c=1.0, $CHCl_3$).
(S)-(6,6'-Dimethoxybiphenyl-2,2'-diyl)bis(di-4-biphenylylphosphin) [(S)-p-Biphenyl-MeOBIPHEP]; Smp. 160-180°; $[\alpha]_D^{20}$ = -39.4 (c=1.0, $CHCl_3$).
(R)-(6,6'-Dimethoxybiphenyl-2,2'-diyl)bis[bis-[4-(trimethylsilyl)-PhenYl]phosphinoxid]; $[\alpha]^{20}{}_D$ = +89.9 (c=0.8,

$CHCl_3$).
(R)-(6,6'-Dimethoxybiphenyl-2,2'-diyl)bis[bis-[4-(trimethylsilyl)-phenyl]phosphin] [(R)-p-TMS-MeOBIPHEP]; Smp. 210.7-211.1°; $[\alpha]^{20}_D$ = +36.7 (c=0.8, $CHCl_3$).

Beispiel 2

Synthese von (R)-(6,6'-Dimethoxybiphenyl-2,2'-diyl)bis[di-(metaterphenyl-5'-yl)phosphin][(R)-3,5-Diphenyl-MeOBIPHEP]

Eine aus 0.24 g (10 mMol) Magnesiumspänen und 2.78 g (9 mMol) 5'-Brom-meta-terphenyl (Chi-Jen Frank Du, Harold Hart, Kwok-Keung Daniel Ng, J. Org. Chem. 1986, 51, 3162) in 40 ml trockenem THF hergestellte Lösung von meta-Terphenyl-5'-ylmagnesiumbromid wurde bei -78° zu 0.45 g (1 mMol) (R)-(6,6'-Dimethoxybiphenyl-2,2'-diyl) bis-(phosphonsäuredichlorid) einfliessen gelassen. Die entstandene homogene Reaktionslösung wurde bei 0° tropfenweise mit einer $NH_4Cl$-Lösung versetzt. Die organische Phase wurde abgetrennt, mit ges. NaCl-Lösung gewaschen, über $MgSO_4$ getrocknet, filtriert und eingedampft. Der Rückstand wurde an 100 g Kieselgel (Hexan/Essigsäureäthylester 10%→50%) chromatographiert. Nach Trocknen am HV (~10 Pa) bei 100° wurden 0.6 g (50%) (R)-(6,6'-Dimethoxybiphenyl-2,2'-diyl)bis[di-(meta-terphenyl-5'-yl)phosphinoxid] als weisses Pulver isoliert; Smp. 181-205°; $[\alpha]^{20}_D$ = +42.4 (c=1.0, $CHCl_3$).

Zu einer Lösung von 0.5 g (0.4 mMol) (R)-(6,6'-Dimethoxybiphenyl-2,2'-diyl)bis[di-(meta-terphenyl-5'-yl)phosphinoxid] in trockenem Xylol und 4 ml trockenem Tributylamin wurden unter Ar und unter Kühlung 2 ml Trichlorsilan getropft. Die Reaktionslösung wurde 3 Stunden unter Ar am Rückfluss gekocht. Nach Abkühlung wurde das Reaktionsgemisch unter Ar via eine Stahlkanüle in 50 ml deoxygenierte 30% NaOH-Lösung eingetragen, wobei die Temperatur bis auf 60-70° anstieg. Das Zweiphasengemisch wurde noch 1 Stunde bei 60-70° gerührt, und nach Abkühlung wurden die Phasen getrennt. Die organische Phase wurde mit Wasser (2 x 50 ml) und mit ges. NaCl-Lösung (2 x 50 ml) gewaschen, über $MgSO_4$ getrocknet, filtriert und am Rotationsverdampfer eingedampft. Nach Trocknung am HV (-10 Pa) bei 80° wurde der Rückstand an Kieselgel chromatographiert (50 g, $CH_2Cl_2$/Hexan, dann $CH_2Cl_2$). Das erhaltene Produkt wurde aus Aethano/$CH_2Cl_2$) umkristallisiert. Nach Trocknen am HV (~10 Pa) bei 100° wurden 0.4 g (R)-(6,6'-Dimethoxybiphenyl-2,2'-diyl)bis[di-(metaterphenyl-5'-yl)phosphin] als weisses Pulver vom Smp. 148-155° isoliert; $[\alpha]^{20}_D$ = -3.5 (c=1.0, $CHCl_3$).

In zum Vorhergehenden analoger Weise wurden folgende Verbindungen hergestellt:

(S)-(6,6'-Dimethoxybiphenyl-2,2'-diyl)bis[di-(meta-terphenyl-5'-yl)-Phosphinoxid]; Smp. 179-203° (chromatographiert); $[\alpha]^{20}_D$ = -43.7 (c=1.0, $CHCl_3$).
(S)-(6,6'-Dimethoxybiphenyl-2,2'-diyl)bis[di-(meta-terphenyl-5'-yl)-phosphin]; Smp. 150-158° (chromatographiert); $[\alpha]^{20}_D$ = +4.0 (c=1.0, $CHCl_3$).

Beispiel 3

In zu den Beispielen 1 oder 2 analoger Weise können folgende Verbindungen hergestellt werden:

(R)-(6,6'-Dimethoxybiphenyl-2,2'-diyl )bis[bis-(3,5-bis(trimethylsilyl)phenyl)Phosphinoxid]; Smp. 258-260° (chromatographiert); $[\alpha]^{20}_D$ = +62.4 (c=1.0, $CHCl_3$) [das für die Grignardreaktion benötigte 1-Brom-3,5-bis(trimethylsilyl) benzol wurde hergestellt nach B.M. Trost, D.J. Murphy, Organometallics 1985, 4, 1143].
(R)-(6,6'-Dimethoxybiphenyl-2,2'-diyl)bis[bis-(3,5-bis(trimethylsilyl)phenyl)phosphin] [(R)-3,5-TMS-MeOBIPHEP]; Smp. 207-209°; $[\alpha]^{20}_D$ = +19.7 (c=1.0, $CHCl_3$).
(S)-(6,6'-Dimethoxybiphenyl-2,2'-diyl)bis[bis-(3,5-bis(trimethylsilyl)phenyl)phosphinoxid]; Smp. 258-260° (chromatographiert); $[\alpha]^{20}_D$ = -62.4 (c=1.0, $CHCl_3$).
(S)-(6,6-Dimethoxybiphenyl-2,2'-diyl)bis[bis-(3,5-bis(trimethylsilyl)phenyl)phosphin][(S)-3,5-TMS-MeOBIPHEP]; Smp. 208-209°; $[\alpha]^{20}_D$ = -19.2 (c=1.0, $CHCl_3$).
(R)-(6,6'Dimethoxybiphenyl-2,2'-diyl)bis[bis-(3,5-bis(triäthylsilyl)phenyl)phospinoxid]; $[\alpha]^{20}_D$ = +31.0 (c=1.0, $CHCl_3$).
(R)-(6,6'-Dimethoxybiphenyl-2,2'-diyl)bis[bis-(3,5-bis(triäthylsilyl)phenyl)phospin]; Smp. 58-59°; $[\alpha]^{20}_D$ = -18,1 (c=1.0, $CHCl_3$).
(S)-(6,6'-Dimethoxybiphenyl-2,2'-diyl)bis[bis-(3,5-bis(triäthylsilyl)phenyl)phosphinoxid]; $[\alpha]^{20}_D$ = -30 (c=1.0, $CHCl_3$).
(S)-(6,6'-Dimethoxybiphenyl-2,2'-diyl)bis[bis-3,5-bis(triäthylsilyl)phenyl)phosphin]; Smp. 58-59°; $[\alpha]^{20}_D$ = +16.5 (c=1.0, $CHCl_3$).
(S)-(6,6'-Dimethoxybiphenyl-2,2'-diyl)bis(diisopropylphosphinoxid); Smp. 194.5-196.5°; $[\alpha]^{20}_D$ = -61.3° (c=1.0,

$CHCl_3$).
(S)-(6,6'-Dimethoxybiphenyl-2,2'-diyl)bis(diisopropylphosphin); Smp. 138-140°; $[\alpha]_D^{20}$ = -18.4° (c=1.0, $CHCl_3$).
(R)-(6,6'-Dimethoxybiphenyl-2,2'-diyl)bis(diisopropylphosphinoxid); Smp. 195-196°; $[\alpha]_D^{20}$ = +62° (c=1.0, $CHCl_3$).
(R)-(6,6'-Dimethoxybiphenyl-2,2'-diyl)bis(diisopropylphosphin); Smp. 140-141°; $[\alpha]_D^{20}$ = +19.7° (c=1.0, $CHCl_3$).
(RS)-(6,6'-Dimethoxybiphenyl-2,2'-diyl)bis(diäthylphosphinoxid); Smp. 255-256°.
(RS)-(6,6'-Dimethoxybiphenyl-2,2'-diyl)bis(diäthylphosphin); Smp. 137°.

Beispiel 4

a) In einer Glove-Box (<1 ppm Sauerstoff) wurden 2,06 mg (0.0063 mMol) Di($\eta^2$-acetato)-($\eta^4$-cycloocta-1,5-dien) ruthenium(II) [B. Heiser et al., Tetrahedron: Asymmetry 2, 51 (1991)] und 5.6 mg (0.0063 mMol) (R)-p-Biphenyl-MeOBIPHEP (hergestellt gemäss Beispiel 1) in 0.7 ml Tetrahydrofuran und 2 ml Aether bei 40° während 16 Stunden gerührt, wobei sich eine gelbe, klare Lösung des Katalysators bildete.

b) In einer Handschuhbox wurde ein 500 ml-Autoklav mit 15.0 g (50.4 mMol) (Z)-2-Acetyl-1-(p-methoxybenzyliden)-1,2,3,4,5,6,7,8-octahydroisochinolin, 170 ml Methanol und der gemäss a) hergestellten Katalysatorlösung beladen. Die Hydrierung wurde bei 100° und 35 bar während 22 Stunden durchgeführt. Der Umsatz betrug 98.5%. Ein 2 g Produkt enthaltender Teil der Hydrierlösung wurde eingedampft und der Rückstand in Diäthyläther gelöst. Zur Abtrennung des Katalysators wurde die Aetherlösung durch ein Kieselgel-Polster filtriert. Eindampfen des Filtrates ergab 1.97 g (S)-2-Acetyl-1-(p-methoxybenzyl)-1,2,3,4,5,6,7,8-octahydroisochinolin als gelbliche Kristalle von 95.9% e.e.

Zur Bestimmung des e.e.-Wertes wurde das Produkt in einem Gemisch von Aethylenglykol und 40% wässriger Kaliumhydroxid-Lösung bei 170° während 18 Stunden hydrolysiert. Das gebildete Amin wurde mit (-)-Camphansäurechlorid in Pyridin/4-Dimethylaminopyridin zum Gemisch der diastereomeren Amide umgesetzt, und letzteres mittels GC analysiert.

Beispiel 5

In zu Beispiel 4 analoger Weise wurde der Katalysator mit (R)-p-TMS-MeOBIPHEP (hergestellt gemäss Beispiel 1) als Ligand hergestellt und die Hydrierung in ebenfalls analoger Weise durchgeführt: 94% Umsatz, 96% e.e.

Beispiel 6

In einer Glove-Box ($O_2$-Gehalt <1 ppm) wurden in einem 50 ml Schlenkrohr 10.14 mg (0.031 mMol) Di($\eta^2$-acetato-($\eta^4$-cycloocta-1,5-dien)ruthenium(II) [B. Heiser et al., Tetrahedron: Asymmetry 2, 51 (1991)] und 13.83 mg (0.031 mMol) [(S)-6,6'-Dimethoxybiphenyl-2,2'- diyl]bis[diisopropylphosphin] (hergestellt gemäss Beispiel 3) in 6 ml Aether und 2 ml THF gelöst und 1.5 Stunden bei 40° gerührt. Es bildete sich eine rote klare Katalysatorlösung. Die Hydrierung erfolgte in einem 500 ml-Autoklav, der mit 8.0 g (31.0 mMol) 3,4,6,11-Tetrahydro-6,11-dioxo-pyridazo[1,2-a] phthalazin-1-carbonsäure, 3.14 g (31.0 mMol) Triäthylamin, 150 ml Methanol und mit der oben hergestellten Katalysatorlösung beladen wurde. Die Hydrierung wurde bei 60°, einem konstanten Druck von 40 bar reinem Wasserstoff und unter intensivem Rühren durchgeführt. Nach 5 Stunden betrug der Umsatz gemäss GC 99.9%. Die gelbe Hydrierlösung wurde am Rotationsverdampfer bei 50°/220 mbar bis auf ein Gewicht von 25 g eingedampft. Zu der verbleibenden Lösung wurden unter Rühren, bei 20-35°, 4.42 ml 25-proz. HCl-Lösung und anschliessend 27 ml Wasser zugetropft. Die (S)-Säure begann bei ca. 25° auszufallen. Die Suspension wurde 1 Stunde bei 20° und 1 Stunde bei 0° gerührt. Nach Filtration und Trocknung erhielt man 7.7 g (96%) (S)-1,2,3,4,6,11-Hexahydro-6,11-dioxo-pyridazo [1,2-b]-phthalazin-1-carbonsäure als fast weisse Kristalle mit einer enantiomeren Reinheit von 96.5% e.e. Der e.e.-Wert wurde durch HPLC auf einer $\alpha$-APG-Säule bestimmt.

## Patentansprüche

**1.** Racemische und optisch aktive Phosphorverbindungen der allgemeinen Formel

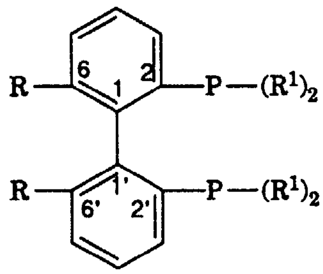

I

worin R $C_{1-5}$- Alkyl oder $C_{1-5}$- Alkoxy bedeutet und $R^1$ $C_{1-5}$- Alkyl, oder mit Phenyl oder Trialkylsilyl substituiertes Phenyl darstellt.

2. Racemische und optisch aktive Phosphorverbindungen der Formel I gemäss Anspruch 1, worin R Methoxy darstellt.

3. Racemische und optisch aktive Phosphorverbindungen der Formel I gemäss einem der Ansprüche 1 oder 2, worin $R^1$ Isopropyl darstellt.

4. (R)- oder (S)-(6,6'-Dimethoxybiphenyl-2,2'-diyl)bis[bis-[4-(trimethylsilyl)phenyl]phosphin].

5. (R)- oder (S)-(6,6'-Dimethoxybiphenyl-2,2'-diyl)bis[bis(3,5-bis(trimethylsilyl)phenyl)phosphin].

6. (R)- oder (S)-(6,6'-Dimethoxybiphenyl-2,2'-diyl)bis(diisopropylphosphin).

7. Komplexe von Phosphorverbindungen der Formel I gemäss Anspruch 1 mit einem Metall der Gruppe VIII.

8. Komplexe gemäss Anspruch 7, worin als Metall der Gruppe VIII Ruthenium, Rhodium oder Iridium enthalten sind.

9. Komplexe gemäss Anspruch 8, worin als Metall der Gruppe VIII Ruthenium oder Rhodium enthalten sind.

10. Verwendung von Phosphorverbindungen der Formel I gemäss Anspruch 1, in Form ihrer Komplexe mit einem Metall der Gruppe VIII, als Katalysator bei asymmetrischen Hydrierungen und für enantioselektiven Wasserstoffverschiebungen in prochiralen, allylischen Systemen.

## Claims

1. Racemic and optically active phosphorus compounds of the general formula

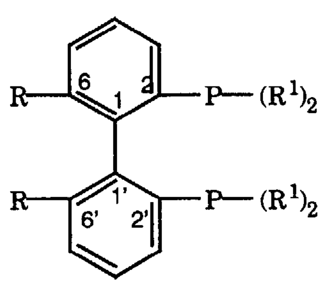

I

wherein R signifies $C_1$-$C_5$-alkyl or $C_1$-$C_5$-alkoxy and $R^1$ represents $C_1$-$C_5$-alkyl or phenyl substituted with phenyl or trialkylsilyl.

2. Racemic and optically active phosphorus compounds of formula I according to claim 1, wherein R represents methoxy.

3. Racemic and optically active phosphorus compounds of formula I according to either claim 1 or 2, wherein $R^1$ represents isopropyl.

**4.** (R)- or (S)-(6,6'-Dimethoxybiphenyl-2,2'-diyl)bis[bis-[4-(trimethylsilyl)-phenyl]phosphine].

**5.** (R)- or (S)-(6,6'-Dimethoxybiphenyl-2,2'-diyl)bis[bis(3,5-bis(trimethylsilyl)phenyl)phosphine].

**6.** (R)- or (S)-(6,6'-Dimethoxybiphenyl-2,2'-diyl)bis(diisopropylphosphine).

**7.** Complexes of phosphorus compounds of formula I according to claim 1 with a metal of Group VIII.

**8.** Complexes according to claim 7, wherein ruthenium, rhodium or iridium is present as the metal of Group VIII.

**9.** Complexes according to claim 8, wherein ruthenium or rhodium is present as the metal of Group VIII.

**10.** The use of phosphorus compounds of formula I according to claim 1 in the form of their complexes with a metal of Group VIII as catalysts in asymmetric hydrogenations and for enantioselective hydrogen displacements in prochiral, allylic systems.

**Revendications**

**1.** Composés racémiques et optiquement actifs du phosphore, de formule générale

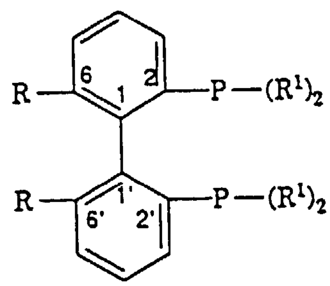

I

dans laquelle R est un groupe alkyle en $C_{1-5}$ ou alcoxy en $C_{1-5}$, et $R^1$ est un groupe alkyle en $C_{1-5}$, ou encore phényle à substitution phényle ou trialkylsilyle.

**2.** Composés racémiques et optiquement actifs du phosphore de formule I selon la revendication 1, dans lesquels R est le groupe méthoxy.

**3.** Composés racémiques et optiquement actifs du phosphore de formule I selon l'une des revendications 1 ou 2, dans lesquels $R^1$ est le groupe isopropyle.

**4.** R)- ou (S)-(6,6'-diméthoxybiphényle-2,2'-diyl)-bis[bis[4-(triméthylsilyl)phényl]phosphine].

**5.** (R)- ou (S)-(6,6'-diméthoxybiphényle-2,2'-diyl)-bis[bis(3,5-bis(triméthylsilyl)phényl)phosphine].

**6.** (R)- ou (S)-(6,6'-diméthoxybiphényle-2,2'-diyl)-bis(diisopropylphosphine).

**7.** Complexes de composés du phosphore de formule I selon la revendication 1 avec un métal du Groupe VIII.

**8.** Complexes selon la revendication 7, dans lesquels le métal du Groupe VIII est le ruthénium, le rhodium ou l'iridium.

**9.** Complexes selon la revendication 8, dans lesquels le métal du Groupe VIII est le ruthénium ou le rhodium.

**10.** Utilisation de composés du phosphore de formule I selon la revendication 1, sous forme de leurs complexes avec un métal du Groupe VIII, en tant que catalyseurs lors des hydrogénations asymétriques, et pour des déplacements d'hydrogène énantio-sélectifs dans les systèmes allyliques prochiraux.